(19) Europäisches Patentamt
European Patent Office
Office européen des brevets

(11) Publication number: 0 061 205
B1

(12) # EUROPEAN PATENT SPECIFICATION

(45) Date of publication of patent specification: 12.03.86

(51) Int. Cl.[4]: C 07 D 499/00, A 61 K 31/43 // C07D205/08, C07F9/65

(21) Application number: 82103773.6

(22) Date of filing: 07.01.80

(60) Publication number of the earlier application in accordance with Art. 76 EPC: 0 013 662

(54) **2-Penem compounds; a method for their preparation and pharmaceutical compositions comprising them.**

(30) Priority: 10.01.79 US 2471
01.08.79 US 62875
05.11.79 US 91610

(43) Date of publication of application: 29.09.82 Bulletin 82/39

(45) Publication of the grant of the patent: 12.03.86 Bulletin 86/11

(84) Designated Contracting States: AT BE CH DE FR GB IT LU NL SE

(56) References cited:
EP-A-0 002 210
EP-A-0 003 960
DE-A-2 819 655

H.R. Pfaendler, "Total Synthesis 9 Hydroxyethylpenem Carboxylic acids" in "Recent Advances in the Chemistry of beta-Lactam Antibiotics"

"Synthesis g Sch 29482 - a novel Antibiotic" in J. of Antimicrobial Chemotherapy" 9, Suppl. C (1982), 1-6

The file contains technical information submitted after the application was filed and not included in this specification

(73) Proprietor: SCHERING CORPORATION
2000 Galloping Hill Road
Kenilworth, New Jersey 07033 (US)

(72) Inventor: McCombie, Stuart Walter
159 Pleasant Valley Way
West Orange New Jersey 07052 (US)

(74) Representative: Ritter, Stephen David et al
Mathys & Squire 10 Fleet Street
London EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the alkali metal salts, and the phthalidyl and pivaloyloxymethyl esters of a compound of formula

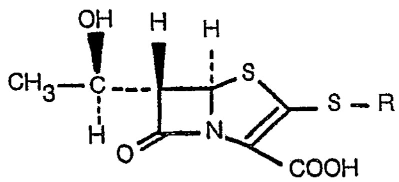

I

in which R is hydroxy-$C_1$—$C_6$ alkyl or carboxy-$C_1$—$C_6$ alkyl, and mixtures thereof with their enantiomers.

The $C_1$—$C_6$ alkyl groups referred to above are exemplified by methyl, ethyl, propyl, butyl, pentyl, hexyl, and the corresponding branched-chain isomers thereof.

The alkali metal salts referred to above preferably are potassium and sodium but may also be lithium, rubidium or cesium salts.

With respect to those in the penem nucleus itself, the configurations at the 5 and 6 positions are of the absolute stereochemistry R and S, respectively. The two hydrogen atoms attached to the 5 and 6 carbon atoms are thus *trans* to one another. The C—8 carbon atom has the 8R stereochemistry, the compounds thus being designated 5R,6S,8R.

The compounds of the present invention may be prepared as their racemic mixtures, *e.g.*, the 5R,6S,8R compound is produced with its enantiomer (mirror image), *i.e.*, a 5S,6R,8S compound, in equal amounts when the starting compound is a racemic mixture. The two enantiomers may be separated by conventional means, *e.g.*, by fractional crystallizations of optically active salt forms, e.g., the salts derived from optically active amino compounds, e.g., (−)-brucine, or (+)- and (−)-ephedrine. Alternatively, the 5R,6S,8R compounds may be produced in their pure enantiomeric form by utilizing optically active starting materials in the synthetic procedure.

The designations of absolute spatial configuration are based on X-ray crystal analysis.

The compounds of this invention possess antibacterial activity of both the gram-positive and gram-negative type. Thus, when tested in standardized microbiological assays, the compounds of this invention are active against such gram-positive organisms as *Staphylococcus epidermidis*, and *Bacillus subtilis*, and such gram-negative organisms as *E. Coli* and *Salmonella* at test levels of 0.1 to 100 ug/ml. Additionally, they show activity against such organisms in the presence of β-lactamase indicating a resistance to these enzymes and are inhibitors of beta-lactamases.

Thus, the present invention includes within its scope pharmaceutical compositions comprising an antibacterially effective amount of a penem as defined above together with a compatible, pharmaceutically acceptable carrier or excipient, and dosage forms, specifically for oral use.

The dosage administered of the penems of this invention is dependent upon the age and weight of the animal species being treated, the mode of administration, and the type and severity of bacterial infection being prevented or reduced. Typically, the dosage administered per day will be in the range of 100—5000 mg, with 500—1000 mg being preferred. For oral administration, the compounds of this invention may be formulated in the form of tablets, capsules, elixirs or the like. Likewise, they may be admixed with animal feed. They may also be applied topically in the form of ointments, both hydrophilic and hydrophobic, in the form of lotions which may be aqueous, non-aqueous or of the emulsion type, or in the form of creams.

The compounds of this invention may be utilized in liquid form such as solutions, suspensions and the like for otic and optic use and may also be administered parenterally via intra-muscular injection.

The compounds of the present invention are prepared by cyclising a compound of the formula

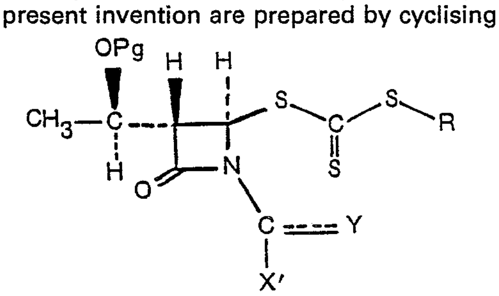

II

wherein the functional group in R is optionally protected, X′ is a protected carboxyl group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisomers was subjected to cyclisation, before or after removing the protecting groups; and isolating the compound of formula I or a mixture thereof with its enantiomer as an alkali metal salt, or converting the cyclisation product to a phthalidyl or pivaloyloxymethy ester or a mixture thereof with its

respective enantiomer; if desired the process including the step of separating a mixture of enantiomers if a mixture containing an enantiomer of the compound of formula II was subjected to cyclisation.

The cyclization is generally conducted at a temperature between 30 to 160°C and preferably at reflux temperatures in an organic solvent such as benzene, toluene or xylene under an inert atmosphere, *e.g.*, nitrogen or argon. Reaction times generally vary from 12—48 hours.

The group Y in the starting material of formula II is a phosphonio group customary for a Wittig reaction, especially a triaryl-, *e.g.* triphenyl- or tri-*p*-methoxyphenyl-, or tri-lower alkyl, *e.g.* tributylphosphonio, or a phosphonato group, e.g. diphenylphosphonato or dimethoxyphosphonato.

The conventional carboxy protecting groups, *e.g.* benzyl, *p*-nitrobenzyl and benzhydryl, can be removed by hydrogenation. Certain hydroxy protecting groups such as trichloroethoxycarbonyl may be removed prior to the carboxy protecting group by deprotection via zinc/acetic acid in a suitable aprotic solvent such as tetrahydrofuran. Most preferably, however, the allyl protecting group will be utilized as carboxy protecting group. This group is most preferably removed by utilizing a suitable aprotic solvent, such as tetrahydrofuran, diethyl ether or methylene chloride, with potassium or sodium 2-ethylhexanoate or 2-ethylhexanoic acid and a mixture of a palladium compound and triphenyl phosphine as the catalyst. This deprotection method is particularly suitable for the sensitive beta-lactam carboxylates of this invention. Use of the potassium or sodium 2-ethylhexanoate provides the corresponding salt, while use of 2-ethylhexanoic acid affords the free carboxy, or hydroxy group.

The starting materials of formula II are preparable by a reaction sequence starting with a compound of the formula

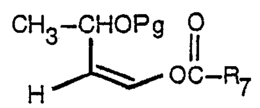

(IV)

wherein $R_7$ is phenyl or an alkyl group containing 1—6 carbon atoms. The hydroxy substituent must be blocked, prior to reaction, with a suitable protecting group, designated Pg, e.g. benzyloxycarbonyl, *p*-nitrobenzyloxycarbonyl, benzhydryloxycarbonyl, allyloxycarbonyl or trichloroethoxycarbonyl. The compound (IV) is reacted with chlorosulfonylisocyanate, followed by a hydrolytic workup, according to procedures disclosed in *Annalen, 1974*, 539, and German Patents 1906401 and 1945549, to provide the intermediate of formula (V)

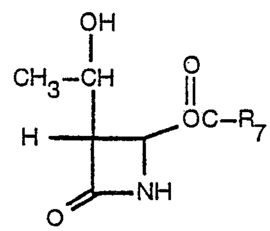

(V)

An alternate method of preparation of the intermediate of formula (V) involves ozone oxidation of a 4-phenyl, 4-methoxyphenyl, or 4-vinylazetidinone having the 1-hydroxyethyl substituent at the 3-position to obtain the 4-carboxylic acid compound. The 4-phenyl and 4-methoxyphenyl compounds are preparable by the process of *Agnew, Chem. Int. Ed. 7*, 172 (1968) while the 4-vinyl compound is preparable from the process of *Canadian J. Chem. 50*, 3196 (1972). The 4-carboxylic acid compound is then treated with lead tetraacetate in an inert solvent to provide the desired intermediate of formula (V). A further alternative route to intermediates similar to those of formula (V) starts with 4-ethylthioazetidin-2-one (preparable according to the procedures of *Liebigs Ann. Chem., 1974* 539—560). This starting material is treated with a suitable amine-protecting group (for the NH function) to afford the 1-protected-4-ethylthioazetidin-2-one. Preferred protecting groups are those such as *t*-butyldimethylsilyl, triethylsilyl, or tetrahydropyranyl, with *t*-butyldimethylsilyl being particularly preferred. Typically, the reaction is conducted in an organic solvent such as dichloromethane or chloroform in the presence of an acid acceptor. The acid acceptor may be an inorganic or organic base, but organic bases such as triethylamine are generally preferred. Typically, temperatures are from 0°C to room temperature, and typical times range from 5—60 minutes, depending upon the nature of the reactants.

The 1-protected-4-ethylthioazetidin-2-one is treated with a strong base to form an anion at the 3-position which is reacted without isolation with acetaldehyde to afford the intermediate of the formula (VI): wherein $R_{20}$ is a nitrogen-protecting group.

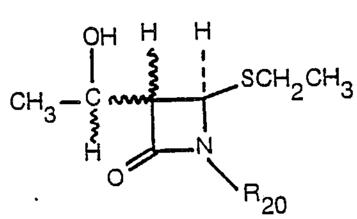

(VI)

The base utilized to form the anion is one such as lithium di-isopropylamine, lithium bis-(trimethylsilyl)amide, *n*-butyl lithium or sec-butyl lithium.

Generally, an anhydrous aprotic solvent such as tetrahydrofuran or ethyl ether is utilized. Preferred temperatures range from −80°C to −60°C during the production of the anion. After addition of the acetaldehyde the reaction mixture may be allowed to warm to room temperature. This reaction produces a mixture of 4 isomers which may be utilized further without separation or which may be optionally separated by chromatography at this stage.

The intermediate of formula (VI) is then treated with a suitable hydroxy-blocking reagent to afford the intermediate of the formula (VI′).

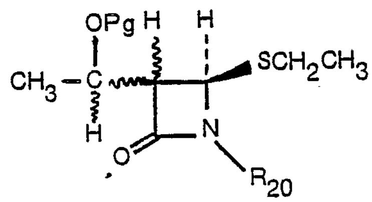

(VI′)

wherein Pg and $R_{20}$ are as hereinbefore defined. Suitable hydroxy protecting groups are those such as 2,2,2-trichloroethoxycarbonyl, 1,1,1-trichloro-2-methyl-2-propoxycarbonyl, *p*-nitrobenzyloxycarbonyl or allyloxycarbonyl, with 2,2,2-trichloroethoxycarbonyl being preferred. Typically, the reaction is conducted in an organic solvent, *e.g.* methylene chloride in the presence of an acid acceptor, *e.g.* triethylamine.

The $R_{20}$-nitrogen-protecting group may then be removed by conventional methods depending upon the exact nature of the $R_{20}$-nitrogen-protecting group utilized. This affords the intermediate of the formula (VII)

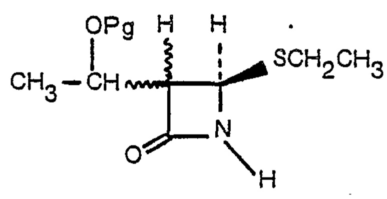

(VII)

Treatment of the intermediate of formula (V) with a nucleophile of the formula (VIII) or treatment of the intermediate of formula (VII) with chlorine, followed immediately by a nucleophile of the formula (VIII)

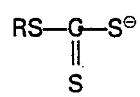

(VIII)

wherein R is as defined for formula (I) and has suitable hydroxy or carboxy protecting groups, affords the compound of formula (IX).

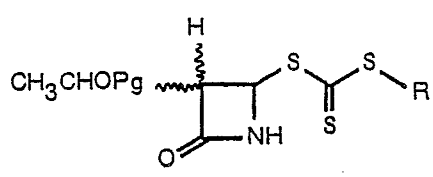

(IX)

A monosubstituted lactam of formula (V) or (VII), will give predominately the *trans* product in the nucleophilic reaction together with a small amount of the *cis* product. Thus, the compounds of formula (IX) produced will have predominately the following relative stereochemistry

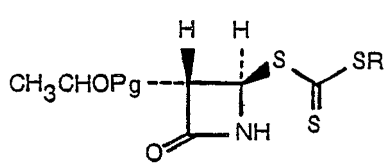

The nucleophile of formula (VIII) is generated *in situ* by reaction of carbon disulfide, the appropriate thiol and a base such as potassium or sodium hydroxide. Generally, the addition of chlorine is at low temperatures (−30 to −10°C), but the subsequent reaction with the nucleophile of formula (VIII) is conducted at slightly higher temperatures, *e.g.* −10 to +10°C. This reaction produces predominately the two *trans* isomers, *e.g.* the compounds differing in their relative stereochemistry at the asymmetric carbon attached at the 3-position of the azetidin-2-one ring. These two isomers are separated by conventional methods, *e.g.* crystallization and/or chromatography, at this stage of the reaction sequence.

In the foregoing procedure (VI → VI′ → VII), if the removal of the $R_{20}$-nitrogen-protecting group and introduction of the protecting group Pg is postponed until after the reaction with chlorine and the nucleophile of formula (VIII), there is produced a product mixture containing predominately the two *cis* isomers of the following formula after appropriate introduction of the Pg-hydroxy-protecting group

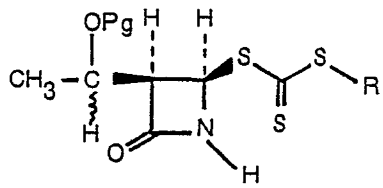

which are then separated by conventional methods, *e.g.* crystallization and/or chromatography, at this stage of the reaction sequence, together with the two *trans* isomers.

The compounds of the above formulae are each produced by the above-described processes in admixture with an equal quantity of their mirror images. Where the pure optically active final products are desired, the intermediates may be resolved by conventional means into their optically active forms. Alternatively, optically active compounds of formula (IX), are preparable by starting from naturally occurring optically active penicillins. One such particularly preferred process comprises:

(a) treatment of an optically active compound of the formula

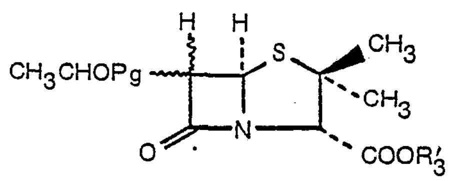

wherein $R_3'$ is a lower alkyl or aralkyl group with elemental chlorine, to afford predominantly the *trans* intermediate of the formula

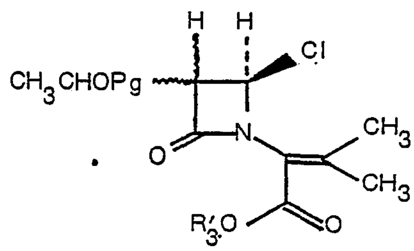

(b) ozonolysis thereof to afford the intermediate of the formula

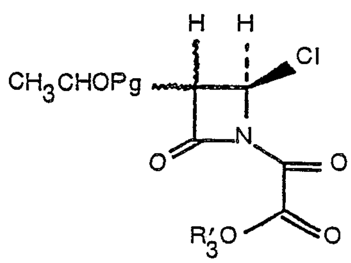

(c) and reaction thereof with a nucleophile of the formula (VIII)

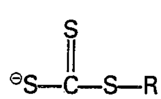 (VIII)

The first step (a) of this process is typically conducted in a suitable organic solvent at temperatures of about −30 to 0°C. Particularly suitable solvents are those such as methylene chloride, chloroform, carbon tetrachloride, toluene and xylene. Preferably, a nitrogen atmosphere is also used. The elemental chlorine is typically added as a solution having concentration of 0.5 to 5 M in a suitable organic solvent, *e.g.* carbon tetrachloride.

The second step (b) of the above described process is typically conducted at low temperatures, *e.g* about −80 to about −40°C in a non-polar, organic solvent. Most preferably, the same solvent is utilized for

this step as for step (a) of the instant process. A particularly suitable solvent is methylene chloride but others, such as chloroform, or xylene, may also be used.

The final step (c) of the above described process may be conducted without isolation and purification of the intermediate. Typically, this reaction is conducted at temperatures of about 0 to 50°C, with room temperatures being most particularly preferred.

The nitrogen of the lactam of formula (IX) is then reacted with an aldehyde X'—CHO to afford the compound of formula (X)

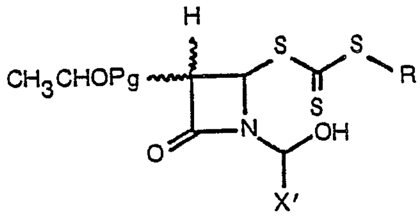

(X)

wherein X' is protected carboxyl. The carboxyl protecting group may be any suitable group such as *p*-nitrobenzyl, benzyl, allyl or benzhydryl. In a highly preferred embodiment, it will be an allylic group so as to provide the possibility to applying neutral conditions when it is removed at the end of the sequence.

This reaction is usually preferably conducted at reflux temperatures in an non-polar aprotic solvent such as tetrahydrofuran or benzene. Reaction times of 2—10 hours are generally typical.

The compound of formula (X) is then treated with a chlorinating or brominating agent, *e.g.* thionyl chloride, methanesulfonyl chloride, thionyl bromide, or phosphorous tribromide in the presence of an equivalent of an acid acceptor, *e.g.* pyridine or triethylamine, so as to afford replacement of the hydroxy group in position α relative to the ring nitrogen.

Suitable solvents are those such as methylene chloride, tetrahydrofuran or benzene. Temperatures of about 0—20°C and reaction times of 10—60 minutes are generally preferred.

This chloride or bromide is then reacted with a suitable phosphine, *e.g.* tri-*p*-methoxyphenyl phosphine, tributylphosphine or most preferably, triphenylphosphine to afford the compound of the formula (II).

Typically, the reaction is conducted at room temperature in a polar aprotic solvent such as hexamethylphosphoramide or dimethylformamide. Reaction times generally vary from about 12—48 hours.

The transformations of compound (IX) to compound (II) are generally according to the procedures described by Woodward, *et. al., Helv. Chim. Acta.*, 55, 408—423 (1972).

Alternatively, in this synthetic procedure, when trimethoxyphosphine or sodium diphenylphosphinate is used in place of *e.g.* triphenylphosphine the corresponding dimethoxyphosphonate or diphenylphosphonate is obtained.

Treatment of this phosphonate with a base such as sodium hydride in a polar solvent (dimethylformamide) results in an unisolated intermediate of the structure

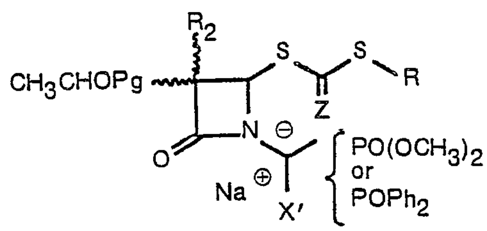

which is also embraced by formula (II).

The phthalidyl or pivaloyloxymethyl esters are prepared by reaction of the corresponding alkali metal salts with chlorophthalide or pivaloyloxymethyl chloride in a solvent such as dimethylformamide. Preferably, a catalytic amount of sodium iodide is added.

The following preparations and Examples illustrate the invention. Throughout these preparations and Examples, "NMR" denotes nuclear magnetic resonance spectra; "rotation" denotes optical rotation of the compounds in a suitable solvent; "MS" denotes mass spectra; "UV" denotes ultraviolet spectra; and "IR" denotes infrared spectra. Chromatography is performed on silica gel unless otherwise noted.

## Example 1

Preparation of Potassium (5R,6S,8R)-6-(1-hydroxyethyl)-2-[(2-hydroxyethyl)thio] penem-3-carboxylate, as a mixture with its enantiomer

The procedure followed in this Example was a repetition of the procedure detailed in steps a) to g) of Example 1 of European Application No. 80810004.4 (from which this Application has been divided out), that is to say:

a. Ethyl [1-(Allyloxycarbonylhydroxymethyl)-3-(1-trichloroethoxycarbonyloxyethyl)-2-azetidinone-4-yl] trithiocarbonate

(i) Isomer I

A mixture of the major isomer of the product thiocarbonate from Preparation A* (2.13 g), allyl glyoxylate hydrate (1.0 g) (from Preparation D)* and benzene (25 ml) is refluxed under argon with a water collector for 20 hours. The solution is cooled, diluted with dichloromethane (70 ml) and washed with water (2 × 100 ml), dried and evaporated to give a thin layer chromatography-pure product as a yellow oil.

(ii) Isomer II

Repetition of the procedure of the foregoing paragraph a(i), using the minor isomer of Preparation A* (1.02 g) and allyl glyoxylate (0.48 g) in 15 ml benzene with 20 hours reflux is prepared the minor isomer product of this example as a yellow oil.

b. Ethyl [1-(Allyloxycarbonylchloromethyl)-3-(1-trichloroethoxycarbonyloxyethyl)-2-azetidinone-4-yl]trithiocarbonate

(i) Isomer I

The Isomer I product of the foregoing paragraph a(i) (2.77 g) in dichloromethane (30 ml) and methanesulfonylchloride (0.87 g) is stirred at 0°C, and triethylamine (0.78 g), added dropwise. After 5 minutes at room temperature, the solution is diluted with dichloromethane, washed with 5% aqueous tartaric acid and sodium bicarbonate, dried and evaporated to give a brown oil, having infrared spectrum vmax (film) at 1770 and 1750—1735 (broad) $cm^{-1}$.

(ii) Isomer II

The procedure of the foregoing paragraph b(i) is followed using the Isomer II product of paragraph a(ii) (1.30 g) in 20 ml dichloromethane with triethylamine (0.29 g and mesyl chloride (0.33 g). The final solution after workup is filtered through 5 g silica gel, washing with dichloromethane. Evaporation gives the product as a yellow oil (1.0 g), pure by thin layer chromatography with infrared spectrum vmax (film) 1770, 1755 and 1735 $cm^{-1}$.

C. Ethyl [1-(Allyloxycarbonyl[triphenylphosphoranyl]methyl)-3-(1-trichloroethoxycarbonyloxyethyl)-2-azetidinone-4-yl]-trithiocarbonate

(i) Isomer I

The crude product of the foregoing paragraph b(i) (2.6 g) is stirred with triphenylphosphine (2.8 g) in dry dimethylformamide (30 ml) at room temperature for 40 hours, then extracted with ether, washing with three portions of water. The solution is then dried over anhydrous magnesium sulfate and evaporated. The residue is chromatographed on silica gel (150 g), eluting with 1:1 dichloromethane:hexane to remove excess triphenylphosphine, then with pure dichloromethane to give recovered starting material. The title product is eluted with 5—10% ether-dichloromethane, and pure fractions pooled, evaporated and dried at high vacuum to afford the title compound having infrared spectrum vmax ($CH_2Cl_2$) at 1750, 1730 and 1690 $cm^{-1}$.

(ii) Isomer II

Using 1.08 g of the Isomer II prepared in the foregoing paragraph b(ii) and triphenylphosphine (0.75 g) in dimethylformamide (15 ml) and repeating the procedure of paragraph c(i) with stirring at room temperature for 72 hours affords the desired Isomer II of the title product as a yellow foam.

d. Allyl *trans*-6-(1-trichloroethoxycarbonylethyl)-2-ethylthiopenem-3-carboxylate

(i) Isomer I

A solution of the Isomer I phosphorane prepared as in paragraph c(i), (0.975 g) in dry toluene (20 ml) is refluxed under argon in an oil bath at 120—125°C for 65 hours, then cooled, diluted with 20 ml hexane and applied to approximately 20 g silica gel. Elution with 1:1 dichloromethane:hexane followed by dichloromethane gives Isomer I of the title compound, which is crystallized from dichloromethane:ether:hexane to give fibrous needles (0.255 g), melting point 123—127°C and having infrared spectrum vmax ($CH_2Cl_2$) at 1795, 1745, 1700 $cm^{-1}$. *Anal.*

Found: C 38.8; H, 3.6; N. 2.9%

$C_{16}H_{18}NO_6S_2Cl_3$ req: C 39.15; H, 3.7; N, 2.8%

---

Footnote

* Preparation A and D as described in European Patent Application No. 80810004.4 (Publication No. 0013662)

(ii) Isomer II

By similar heating of the Isomer II of the phosphorane as prepared in paragraph c(ii) (0.90 g) in toluene (10 ml) for 36 hours and plate chromatography, there is obtained Isomer II of the title product as a yellow oil having infrared spectrum vmax ($CH_2Cl_2$) at 1790, 1750, 1705 $cm^{-1}$.

e. Allyl *trans*-6-(1-hydroxyethyl)-2-ethylthiopenem-3-carboxylate

(i) Isomer I

Isomer I of paragraph d(i) (0.175 g) is stirred at 25°C for 1 hour with activated zinc dust (0.05 g) in acetic acid (1 ml) and tetrahydrofuran (3 ml). The mixture is diluted with excess dichloromethane and washed with water, aqueous sodium bicarbonate and aqueous sodium chloride, dried and evaporated. The residue is purified on a preparative thin layer chromatography plate eluting with 20% ether-dichloromethane and crystallized from ether-hexane to afford Isomer I of allyl *trans*-6-(1-hydroxyethyl)-2-ethylthiopenem-3-carboxylate, melting point 65—66°C having infrared spectrum vmax ($CH_2Cl_2$) at 3250, 1790 and 1705 $cm^{-1}$.

(ii) Isomer II

In a manner similar to that of paragraph e (i), 0.15 g of Isomer II of paragraph d (ii) is deprotected in tetrahydrofuran-acetic acid with zinc dust, and chromatographed on a thin layer chromatography plate to obtain Isomer II of allyl *trans*-6-(1-hydroxyethyl)-2-ethylthiopenem-3-carboxylate, which is crystallized from etherhexane as cream prisms, melting point 86—88°C, having infrared spectrum vmax ($CH_2Cl_2$) at 3300, 1795 and 1700 $cm^{-1}$.

f. Potassium trans-6-(1-hydroxyethyl)-2-ethylthiopenem-3-carboxylate

(i) Isomer I

A solution of Isomer I of allyl *trans*-6-(1-hydroxyethyl)-2-ethylthiopenem-3-carboxylate (52 mg) and 0.5m potassium-2-ethylhexanoate (0.36 ml) in 1.2 ml ethyl acetate and 0.8 ml dichloromethane is stirred with addition of 3 mg triphenylphosphine and 5 mg of tetrakis (triphenylphosphine) palladium- (O), under argon. After a few minutes, precipitation of product occurs and after 30 minutes, excess ethyl acetate is added and the precipitate centrifuged. Washing with ether and drying at high vacuum gives, as a yellowish powder, the title product having infrared spectrum vmax (nujol) at 3300, 1775 and 1600 $cm^{-1}$. By X-ray crystallography of the starting materials the stereochemistry of this product is designated 5R,6S,8S, together with its enantiomer.

(ii) Isomer II

The procedure of the foregoing paragraph is repeated using Isomer II of allyl *trans*-6-(1-hydroxyethyl)-2-ethylthiopenem-3-carboxylate (40 mg), 2 ml of 1:1 ethyl acetate:dichloromethane, 0.26 ml of 0.5m potassium-2-ethylhexanoate, 3 mg triphenylphosphine and 5 mg palladium complex. After 20 minutes, ether (2 ml) is added gradually and the product centrifuged and dried under high vacuum to give the title product as a cream powder having infrared spectrum vmax (nujol) at 3400, 1780 and 1605$cm^{-1}$. By X-ray crystallography of the starting materials the stereochemistry of this product is designated 5R,6S,8R, together with its enantiomer.

Thus in the present Example there was carried out the procedure as described above using appropriate starting materials to produce the title compound.

## Example 2

Preparation of Potassium (5R,6S,8R)-6-(1-hydroxyethyl)-2-[(2-hydroxyethyl)thiol]penem-3-carboxylate

The procedure followed in this example was a repitition of the procedure detailed in steps A to F of Example 3 of European Application No. 80810004.4, that is to say:

A. To a solution of 7.7 g (3S,4R,5R)-3-[1-trichloroethoxycarbonyloxyethyl]-4-[(ethylthio)-carbonothioylthio]-azetidin-2-one in 90 ml benzene is added 3.5 g allyl glyoxalate. Under nitrogen this mixture is slowly azeotroped for 24 hours. (The reaction is followed by thin layer chromatography eluting with 10% ethyl ether/methylene chloride). Approximately 2.0 ml additional allylglyoxalate is added and the reaction is azeotroped for 10 additional hours. The reaction is then cooled and 150 ml benzene is added. The resultant solution is washed 5 times with 50 ml portions of water. The solution is dried over anhydrous sodium sulfate and the solvents removed under vacuum. Then, 50 ml toluene is added and removed 3 times under high vacuum to afford as the product 9.2 g crude allyl [(3S,4R,5R)-3-(1-trichloroethoxycarbonyloxyethyl)-4-[(ethylthio)carbonothioylthio]-2-azetidinone-1-yl]-2-hydroxyacetate.

Rotation: $[\alpha]^{26}_{D} = +46.9°$ (0.2% in ethanol)

NMR: δ = 6.07—6.21, 1H; δ = 4.76, 2H, s; δ = 3.17—3.52, 3H; δ = 1.22—1.54, 6H.

B. To a solution of 9.0 g crude allyl [(3S,4R,5R)-3-(1-trichloroethoxycarbonyloxyethyl)-4-[(ethylthio)carbonothioylthio]-2-azetidinone-1-yl]-2-hydroxyacetate in 125 ml dry methylene chloride at 0°C is added 2.8 g methylsulfonyl chloride followed by 2.5 g triethylamine. The reaction is followed by a thin layer chromatography eluting with 5% ethyl ether/methylene chloride. After stirring 45 minutes, 125 ml methylene chloride is added. The reaction is then washed once with cold 10% phosphoric acid solution,

once with water, and once with cold dilute sodium bicarbonate solution and then twice with water. The solution is dried over anhydrous sodium sulfate and the solvents are removed under vacuum. The crude product is chromatographed on coarse silica gel with 20% hexane/chloroform to afford 6.9 g allyl [(3S,4R,5R)-3-(1-trichloroethoxycarbonyloxyethyl)-4-[(ethylthio)carbonothioylthio]-2-azetidinone-1-yl]-2-chloroacetate.

IR: 1760—1800 $cm^{-1}$ ($CDCl_3$)
NMR: δ = 6.23—6.29, 1H; δ = 4.72, 2H, s; δ = 1.24—1.56, 6H; ($CDCl_3$).

C. To a solution of 6.9 g allyl [(3S,4R,5R)-3-(1-trichloroethoxycarbonyloxyethyl)-4-[(ethylthio)-carbonothioylthio]-2-azetidinone]-yl]-2-chloroacetate in 90 ml dimethylformamide at 0°C is added 4.7 g triphenylphosphine. The reaction is allowed to warm to ambient and is stirred 40 hours. (Completion of reaction is determined by thin layer chromatography eluting with methylene chloride). An additional 780 mg triphenylphosphine is added and the reaction stirred at ambient temperature. After 40 hours, the reaction mixture is poured into 300 ml ethyl ether and washed twice with brine solution and 5 times with water. The solvent is dried over anhydrous sodium sulfate and removed under vacuum. The crude product is chromatographed on coarse silica gel with methylene chloride to afford 6.1 g allyl [(3S,4R,5R)-3-(1-trichloroethoxycarbonyloxyethyl)-4-[(ethylthio)carbonothioylthio]-2-azetidinone-1-yl]-2-triphenylphosphine acetate.

Rotation: $[\alpha]^{26}_D = +77.0°$
IR: 1760—1780 $cm^{-1}$ (chloroform solution).
NMR: δ = 6.3—6.4, 1H, δ = 4.70, 2H, s; δ = 1.16—1.49, 6H ($CDCl_3$).

D. A solution of 6.1 g allyl [(3S,4R,5R)-3-(1-trichloroethoxycarbonyloxyethyl)-4-[(ethylthio)-carbonothioylthio]-2-azetidinone-1-yl]-2-triphenylphosphine acetate in 400 ml toluene is refluxed under nitrogen for 22 hours. (Reaction is followed by thin layer chromatography eluting with 5% ethyl acetate/toluene.) The toluene is then removed under high vacuum and the reaction mixture is chromatographed on coarse silica gel with toluene, changing to 10% ethyl acetate/toluene. A mixture of 1.5 g reaction product is isolated which is rechromatographed and then purified by high pressure liquid chromatography with 2% ethyl acetate/toluene to afford 1.18 g allyl (5R,6S,8R)-2-ethylthio-6-[1-trichloroethoxycarbonyloxyethyl]-penem-3-carboxylate and 240 mg allyl (5,6-cis)-2-ethylthio-6-[trichloroethoxycarbonyloxyethyl]penem-3-carboxylate.

5R,6S,8R Rotation: $[\alpha]^{26}_D = +172.8°$ (0.25% in ethanol).
5,6-cis Rotation: $[\alpha]^{26}_D = +156.4°$ (0.45% in ethanol).

E. To a solution of 1.18 g allyl (5R,6S,8R)-2-ethylthio-6-[1-trichloroethoxycarbonyloxyethyl]penem-3-carboxylate in 9.0 ml tetrahydrofuran under nitrogen is added 3 ml acetic acid and 500 mg activated zinc powder. The reaction is stirred for 2 1/2 hours during which time additional 400 mg zinc metal is added in two portions. The reaction is followed by thin layer chromatography eluting with 5% ethyl acetate/toluene. The reaction mixture is then filtered and 150 ml methylene chloride added. After washing twice with water, 3 times with cold 3% sodium bicarbonate solution and twice with brine solution, the solution is dried over anhydrous sodium sulfate. Removal of the solvents under vacuum affords 720 mg allyl (5R,6S,8R)-2-ethylthio-6-(1-hydroxyethyl)-penem-3-carboxylate.

Similarly 220 mg allyl (5,6-cis)-2-ethylthio-6-[trichloroethoxycarbonyloxyethyl]penem-3-carboxylate is converted by above procedure to yield 130 mg allyl (5,6-cis)-2-ethylthio-6-(1-hydroxyethyl)penem-3-carboxylate.

F. To a solution of 700 mg allyl (5R,6S,8R)-2-ethyl-thio-6-(1-hydroxyethyl)penem-3-carboxylate in 4 ml methylene chloride and 8 ml ethyl acetate under nitrogen is added 46.6 mg triphenylphosphine. To this is added 4.86 ml 0.5 molar potassium-2-ethylhexanoate in ethyl acetate. Then, 51.1 mg tetrakis(triphenylphosphine)palladium-(0) is added and the solution is stirred for 15 minutes. An additional 100 mg triphenyl phosphine and 25 mg tetrakis(triphenylphosphine)palladium-(0) is added, followed by 10 ml ethyl ether. The product slowly precipitates and after 1 hour the solution is filtered and washed with ethyl acetate and ethyl ether, to afford 45 mg potassium (5R,6S,8R)-2-ethylthio-6-(1-hydroxyethyl)penem-3-carboxylate. To the mother liquor is added 20 ml ethyl ether. After refrigeration overnight, a second crop of crystals is filtered to yield an additional 90 mg of the potassium sal.

Thus in the present Example the above procedure was carried out using appropriate starting materials to produce the title compound.

**Claims for the Contracting States: BE CH DE FR GB IT LU NL SE**

1. An alkali metal salt, pivaloyloxymethyl or phthalidyl ester of a compound of formula

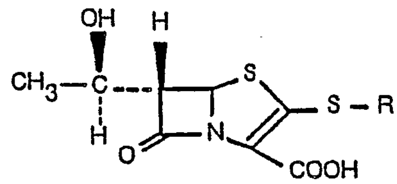

in which R is hydroxy-$C_1$—$C_6$ alkyl or carboxy-$C_1$—$C_6$ alkyl, and mixtures thereof with their enantiomers.

2. An alkali metal salt as defined in Claim 1, being a sodium of potassium salt.

3. Sodium or potassium (5R,6S,8R)-6-(1-hydroxyethyl)-2-(2-hydroxyethylthio) penem-3-carboxylate.

4. A process of preparing an alkali metal salt, pivaloyloxymethyl or phthalidyl ester of a compound of the formula.

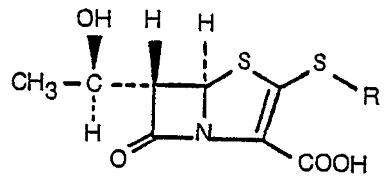

in which R is a hydroxy-$C_1$—$C_6$ alkyl group or a carboxy-$C_1$—$C_6$ alkyl group, or mixtures thereof with their enantiomers, which is characterized by cyclising a compound of the formula

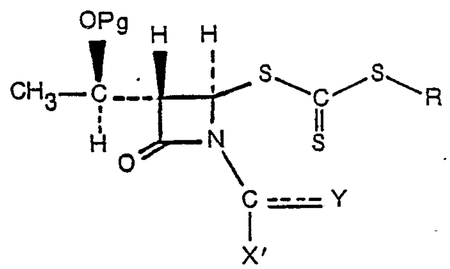

in which the functional group in R is optionally protected, in which X' is a protected carboxyl group, Pg is a hydroxy protecting group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisiomers was subjected to cyclisation, before or after removing the protecting groups; and isolating the compound I or a mixture thereof with its enantiomer as an alkali metal salt, or converting the cyclisation product to a phthalidyl or pivaloyloxymethyl ester or a mixture thereof with its respective enantiomer; if desired the process including the step of separating a mixture of enantiomers if a mixture containing an enantiomer of the compound of formula II was subjected to cyclisation.

5. A pharmaceutical composition comprising a compound as defined in any one of Claims 1 to 3, as an active ingredient.

6. A pharmaceutical composition comprising a compound as defined in Claim 3 as an active ingredient.

**Claims for the Contracting State: AT**

1. A process for preparing an alkali metal salt, or pivaloyloxymethyl, or phthalidyl ester of a compound of the formula

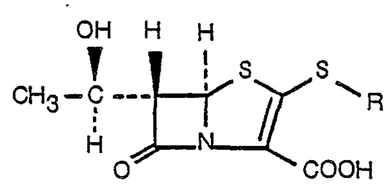

in which R is a hydroxy-$C_1$—$C_6$ alkyl group or a carboxy-$C_1$—$C_6$ alkyl group, and mixtures thereof with their

respective enantiomers which is characterized by cyclising a compound of the formula

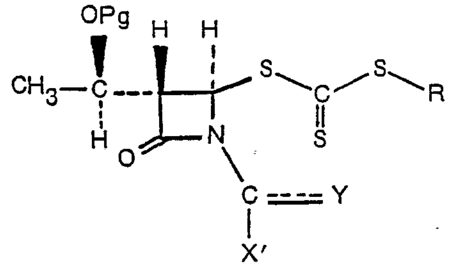

in which any functional group in R is optionally protected, in which X′ is a protected carboxyl group, Pg is a hydroxy protecting group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisiomers was subjected to cyclisation, before or after removing the protecting groups; and isolating the compound of formula I or a mixture thereof with its enantiomer as an alkali metal salt, or converting the cyclisation product to the phthalidyl or pivaloyloxymethyl ester or a mixture thereof with its respective enantiomer; if desired the process including the step of separating a mixture of enantiomers if a mixture containing an enantiomer of the compound of formula II was subjected to cyclisation.

2. A process according to Claim 1, in which the group Y in the compound of formula II is a triaryl- or trialkylphosphonio group.

3. A process according to Claim 2, in which Y is triphenylphosphonio.

4. A process according to any one of Claims 1 to 3, in which the cyclisation is carried out at between 30°C and 160°C in an inert organic solvent.

5. A process according to any one of Claims 1 to 4, wherein a sodium or potassium salt is produced.

6. A process according to any one of Claims 1 to 4, wherein the compound sodium or potassium (5R,6S,8R)-6-(1-hydroxy-ethyl)-2-(2-hydroxyethylthio)-3-carboxylate is produced.

7. A process for preparing a pharmaceutical composition which comprises mixing an alkali metal salt or phthalidyl or pivaloyloxymethyl ester as defined in Claim 1, with a pharmaceutical carrier or excipient.

8. A process for preparing a pharmaceutical composition according to Claim 7, which comprises using an alkali metal salt of a compound of formula I as an active ingredient.

9. A process for preparing a pharmaceutical composition according to Claim 8 in which the alkali metal salt is sodium or potassium (5R,6S,8R)-6-(1-hydroxyethyl)-2-(2-hydroxyethylthio)-3-carboxylate.

**Revendications pour les états contractants: BE CH DE FR GB IT LU NL SE**

1. Sel de métal alcalin, pivaloyloxyméthyl ou phtalidyl ester d'un composé de formule

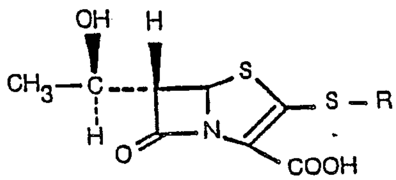

où R est un hydroxyalcoyle $C_1$—$C_6$ ou un carboxyalcoyle $C_1$—$C_6$ et leurs mélanges avec leurs énantiomères.

2. Sel de métal alcalin tel que défini à la revendication 1, qui est un sel de sodim ou de potassium.

3. (5R,6S,8R)-6-(1-hydroxyéthyl)-2-(2-hydroxyéthylthio)pénèm-3-carboxylate de sodium ou de potassium.

4. Procédé de préparation d'un sel de métal alcalin, le pivaloyloxyméthyl ou phtalidyl ester d'un composé de formule

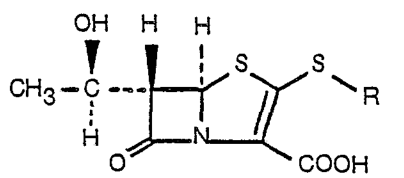

où R est un groupe hydroxyalcoyle $C_1$—$C_6$ ou un groupe carboxyalcoyle $C_1$—$C_6$ ou leurs mélanges avec leurs énantiomères, qui est caractérisé par la cyclisation d'un composé de formule

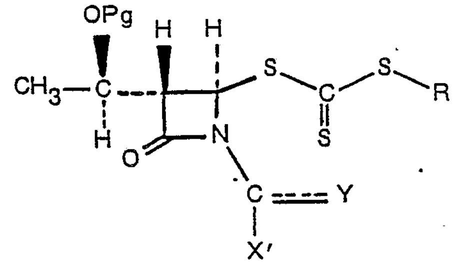

où le groupe fonctionnel dans R est éventuellement protégé, où X' est un groupe carboxyle protégé, Pg est un groupe hydroxy protecteur et Y est un groupe phosphonio étant doublement lié à l'atome de carbone adjacent ou un groupe phosphonato avec une simple liaison à l'atome de carbone adjacent, dont la charge négative est compensée par la présence d'un cation ou un mélange de stéréoisomères le contenant; la séparation d'un mélange de diastéréoisomères, si un mélange contenant des diastéréoisomères a été soumis à la cyclisation, avant ou après enlèvement des groupes protecteurs;
et l'isolement du composé I ou son mélange avec son énantiomère sous la forme d'un sel d'un métal alcalin, ou la conversion du produit de cyclisation en un phtalidyl ou pivaloyloxyméthyl ester ou son mélange avec son énantiomère respectif, si on le souhaite le procédé comprenant l'étape de séparer un mélange d'énantiomères si un mélange contenant un énantiomère du composé de formule II a été soumis à une cyclisation.

5. Composition pharmaceutique comprenant un composé tel que défini selon l'une des revendications 1 à 3, en tant qu'ingrédient actif.

6. Composition pharmaceutique comprenant un composé tel que défini à la revendication 3 en tant qu'ingrédient actif.

**Revendications pour l'Etat Contractant: AT**

1. Procédé du préparation d'un sel de métal alcalin, ou pivaloyloxyméthyl ou phtalidyl ester d'un composé de formule

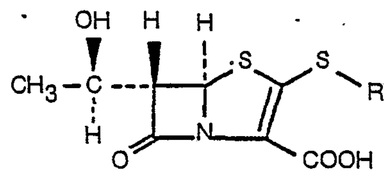

où R est un groupe hydroxyalcoyle $C_1$—$C_6$ ou un groupe carboxyalcoyle $C_1$—$C_6$, et leurs mélanges avec leurs énantiomères respectifs qui est caractérisé par la cyclisation d'un composé de formule

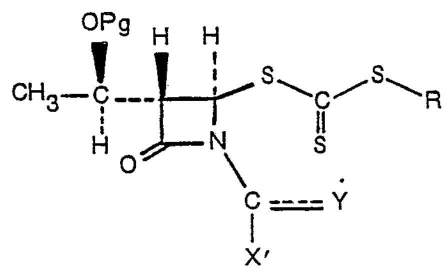

où tout groupe fonctionnel en R est éventuellement protégé, où X' est un groupe carboxyle protégé, Pg est un groupe hydroxy protecteur et Y est un groupe phosphonio étant doublement lié à l'atome de carbone adjacent ou un groupe phosphonato avec une simple liaison à l'atome de carbone adjacent, dont la charge négative est compensée par la présence d'un cation, ou un mélange de stéréoisomères le contenant; la séparation d'un mélange de diastéréoisomères, si un mélange contenant des diastéréoisomères a été soumis à la cyclisation, avant ou après enlèvement des groupes protecteurs;
et l'isolement du composé de formule I ou son mélange avec son énantiomère sous la forme d'un sel d'un métal alcalin, ou la conversion du produit de cyclisation en phtalidyl ou pivaloyloxyméthyl ester ou son mélange avec son énantiomère respectif; si on le souhaite le procédé comprenant l'étape de séparer un mélange d'énantiomères si un mélange contenant un énantiomère du composé de formule II a été soumis à une cyclisation.

2. Procédé selon la revendication 1, où le groupe Y dans le composé de formule II est un groupe triaryl ou trialkylphosphonio.

3. Procédé selon la revendication 2, où Y est triphénylphosphonio.

4. Procédé selon l'une des revendications 1 à 3, où la cyclisation est effectuée entre 30°C et 160°C dans un solvant organique inerte.

5. Procédé selon l'une des revendications 1 à 4, où un sel de sodium ou de potassium est produit.

6. Procédé selon l'une des revendications 1 à 4, où le composé (5R,6S,8R)-6-(1-hydroxyéthyl)-2-(2-hydroxyéthylthio)-3-carboxylate de sodium ou de potassium est produit.

7. Procédé de préparation d'une composition pharmaceutique qui consiste à mélanger un sel d'un métal alcalin ou un phtalidyl ou pivaloyloxyméthyl ester tel que défini à la revendication 1, avec un support ou excipient pharmaceutique.

8. Procédé de préparation d'une composition pharmaceutique selon la revendication 7 qui consiste à utiliser un sel d'un métal alcalin d'un composé de formule I comme ingrédient actif.

9. Procédé de préparation d'une composition pharmaceutique selon la revendication 8 où le sel de métal alcalin est le (5R,6S,8R)-6-(1-hydroxyéthyl)-2-(2-hydroxyéthylthio)-3-carboxylate de sodium ou de potassium.

**Patentansprüche für die Vertragsstaaten: BE CH DE FR GB IT LU NL SE**

1. Alkalimetallsalz, Pivaloyloxymethyl- oder Phthalidylester einer Verbindung der Formel

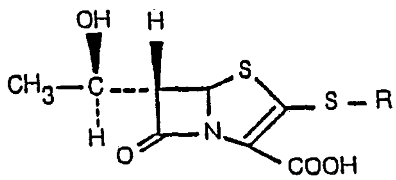

worin R Hydroxy-$C_1$—$C_6$-alkyl oder Carboxy-$C_1$—$C_6$-alkyl bedeutet, und deren Mischungen mit ihren Enantiomeren.

2. Alkalimetallsalz nach Anspruch 1, das ein Natrium- oder Kaliumsalz ist.

3. Natrium oder Kalium (5R,6S,8R)-6-(1-hydroxyethyl)-2-(2-hydroxyethyl-thio)-penem-3-carboxylat.

4. Verfahren zur Herstellung eines Alkalimetallsalzes, Pivaloyloxymethyl- oder Phthalidylesters einer Verbindung der Formel

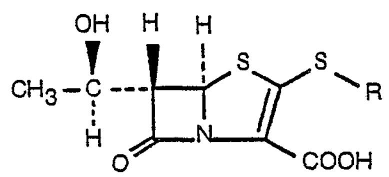

worin R eine Hydroxy-$C_1$—$C_6$-alkyl-Gruppe oder eine Carboxy-$C_1$—$C_6$-alkyl-Gruppe bedeutet, oder deren Mischungen mit ihren Enantiomeren, dadurch gekennzeichnet, daß eine Verbindung der Formel

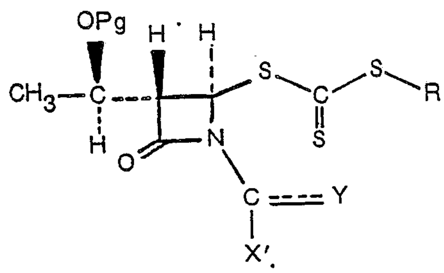

in welcher die funktionelle Gruppe in R gegebenenfalls geschützt ist, in welcher X' eine geschützte Carboxylgruppe, Pg eine Hydroxyschutzgruppe und Y eine an das benachbarte Kohlenstoffatom doppelt gebundene Phosphoniogruppe oder eine Phosphonatogruppe mit einer Einfachbindung an das benachbarte Kohlenstoffatom, dessen negative Ladung durch die Gegenwart eines Kation kompensiert ist, bedeuten, oder eine dieselbe enthaltende Mischung von Stereoisomeren cyclisiert wird, ein Gemisch der Diastereomeren, wenn eine Diastereomere enthaltende Mischung der Cyclisierung unterworfen wurde, vor oder nach dem Entfernen der Schutzgruppen getrennt wird und die Verbindung der Formel I oder eine Mischung derselben mit ihrem Enantiomer als Alkalimetallsalz isoliert wird oder das Cyclisierungsprodukt in den Phthalidyl- oder Pivaloyloxymethylester oder eine Mischung desselben mit seinem entsprechenden Enantiomer umgewandelt wird.

5. Pharmazeutische Zusammensetzung, enthaltend als einen Wirkstoff eine der in einem der Ansprüche 1 bis 3 definierten Verbindungen.

6. Pharmazeutische Zusammensetzung, enthaltend als einen Wirkstoff eine der in Anspruch 3 definierten Verbindung.

**Patentansprüche für den Vertragsstaat: AT**

1. Verfahren zur Herstellung eines Alkalimetallsalzes oder Pivaloyloxymethyl- oder Phthalidylesters einer Verbindung der Formel

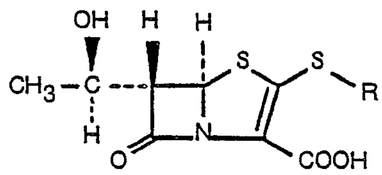

worin R eine Hydroxy-$C_1$—$C_6$-alkyl-Gruppe oder eine Carboxy-$C_1$—$C_6$-alkyl-Gruppe bedeutet, und deren Mischungen mit ihren entsprechenden Enantiomeren, dadurch gekennzeichnet, daß eine Verbindung der Formel

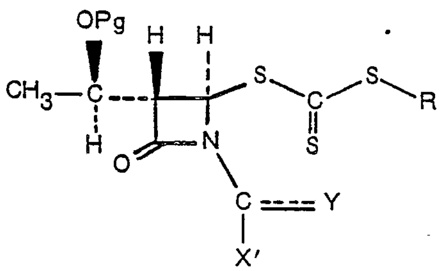

in welcher eine funktionelle Gruppe in R gegebenenfalls geschützt ist, in welcher X' eine geschützte Carboxylgruppe, Pg eine Hydroxyschutzgruppe und Y eine an das benachbarte Kohlenstoffatom doppelt gebundene Phosphoniogruppe oder eine Phosphonatogruppe mit einer Einfachbindung an das benachbarte Kohlenstoffatom, dessen negative Ladung durch die Gegenwart eines Kation kompensiert ist, bedeuten, oder eine dieselbe enthaltende Mischung von Stereoisomeren cyclisiert wird, ein Gemisch der Diastereomeren, wenn eine Diastereomere enthaltende Mischung der Cyclisierung unterworfen wurde, vor oder nach dem Entfernen der Schutzgruppen getrennt wird und die Verbindung der Formel I oder eine Mischung derselben mit ihrem Enantiomer als Alkalimetallsalz isoliert wird oder das Cyclisierungsprodukt in den Phthalidyl- oder Pivaloyloxymethylester oder eine Mischung desselben mit seinem entsprechenden Enantiomer umgewandelt wird.

2. Verfahren nach Anspruch 1, dadurch gekennzeichnet, daß die Gruppe Y in der Verbindung der Formel II eine Triaryl- oder Trialkylphosphonio-Gruppe ist.

3. Verfahren nach Anspruch 2, dadurch gekennzeichnet, daß Y Triphenylphosphonio ist.

4. Verfahren nach einem der Ansprüche 1 bis 3, dadurch gekennzeichnet, daß die Cyclisierung zwischen 30°C und 160°C in einem inerten organischen Lösungsmittel durchgeführt wird.

5. Verfahren nach einem der Ansprüche 1 bis 4, dadurch gekennzeichnet, daß ein Natrium- oder Kaliumsalz hergestellt wird.

6. Verfahren nach einem der Ansprüche 1 bis 4, dadurch gekennzeichnet, daß die Verbindung Natrium- oder Kalium(5R,6S,8R)-6-(1-hydroxyethyl)-2-(2-hydroxyethyl-thio)-3-carboxylat hergestellt wird.

7. Verfahren zur Herstellung einer pharmazeutischen Zusammensetzung, dadurch gekennzeichnet, daß ein Alkalimetallsalz oder Phthalidyl- oder Pivaloylmethylester nach Anspruch 1 mit einem pharmazeutischen Träger oder Hilfsstoff vermischt wird.

8. Verfahren zur Herstellung einer pharmazeutischen Zusammensetzung nach Anspruch 7, dadurch gekennzeichnet, daß ein Alkalymetallsalz einer Verbindung der Formel I als Wirkstoff verwendet wird.

9. Verfahren zur Herstellung einer pharmazeutischen Zusammensetzung nach Anspruch 8, dadurch gekennzeichnet, daß das Alkalimetallsalz Natrium- oder Kalium(5R,6S,8R)-6-(1-hydroxyethyl)-2-(2-hydroxyethylthio)-3-carboxylat ist.